# EUROPÄISCHE PATENTSCHRIFT

(11) **EP 1 611 865 B1**
(45) Veröffentlichungstag und Bekanntmachung des Hinweises auf die Patenterteilung: **13.06.2012**
(21) Anmeldenummer: 05013773.6
(22) Anmeldetag: 27.06.2005
(51) Int. Cl.: A61C 1/18, A61C 1/12, A61B 17/16

(54) **Medizinisches Winkelstück**
Medical contra-angle handpiece
Contre-angle médical

(30) Priorität: 28.06.2004 AT 10902004
(43) Veröffentlichungstag der Anmeldung: 04.01.2006
(73) Patentinhaber: W & H Dentalwerk Bürmoos GmbH, 5111 Bürmoos (AT)
(72) Erfinder: Schatz, Norbert, 5111 Bürmoos (AT)

(56) Entgegenhaltungen:
- WO-A-03/105713
- US-A- 4 568 642
- US-A- 5 169 312
- US-A- 5 902 107

## Beschreibung

Die vorliegende Erfindung betrifft medizinische, insbesondere dentale Winkelstücke zum Antrieb eines mit dem Winkelstück verbundenen Werkzeugs und mit im Winkelstück angeordneten Wellen zur Übertragung der Antriebsbewegung auf das Werkzeug.

Ein derartiges Winkelstück ist zum Beispiel aus der AT 408.514 B bekannt. Im Gegensatz zu den geraden Handstücken weist die Außenhülse der Winkelstücke eine Biegung auf, die bei Winkelstücken der Anmelderin bevorzugt im Bereich von 18° - 21° liegt. Für den Anwender bringt diese Biegung ergonomische Vorteile und erleichtert die Sicht auf die Behandlungsstelle. Im Inneren der Außenhülse des Winkelstücks sind unter anderem die Antriebswellen angeordnet, die die Antriebsbewegung einer Antriebseinheit, bevorzugt eines E-Motors, auf das Werkzeug übertragen.

Der Nachteil bei diesen Winkelstücken ist, daß zur Überbrückung der Biegung der Außenhülse die Antriebswellen im Bereich der Biegung an ihren jeweiligen Enden durch Zahnräder miteinander verbunden werden müssen. Die Herstellung und Montage der Wellen mit den Zahnrädern ist kostenintensiv. Während des Betriebs kommt es durch die Reibung zwischen den kämmenden Zahnrädern weiters zu Verlusten bei der Übertragung des Drehmoments und es entsteht ein zusätzliches Laufgeräusch, das von Anwender und Patienten als störend empfunden wird.

Das Patent US 4,568,642 offenbart ein Winkelstück mit einer geraden, einstückigen Welle, die in einem geraden Hals- und Griffteil aufgenommen ist.

Das Patent US 5,169,312 offenbart ein Winkelstück gemäß dem Oberbegriff des Anspruchs 1 mit einem Griffteil, eine gewinkelt dazu angeordneten Halsteil und einer aus einem Metallkabel bestehenden, flexiblen und gebogenen Welle.

Das Patent US 5,902,107 offenbart ein Winkelstück-Aufsatz für prophylaktische Anwendungen mit einem ziehharmonika-ähnlichen Abschnitt und einer darin aufgenommenen, biegbaren Welle, so dass der Kopf des Winkelstück-Aufsatz verstellbar ist.

Der vorliegenden Erfindung liegt daher die Aufgabe zugrunde ein Winkelstück derart weiter zu entwickeln, daß unter Beibehaltung der Biegung der Außenhülse und der dadurch erreichten Vorteile eine verbesserte Übertragung des Drehmoments mit geringeren Verlusten, eine Reduktion der Lärmemission und eine Verringerung des Herstellungs- und Montageaufwands ermöglicht wird.

Diese Aufgabe wird gemäß der vorliegenden Erfindung durch ein Winkelstück mit den Merkmalen des Anspruchs 1 gelöst.

Überraschender Weise konnte somit ein Winkelstück mit einer Welle mit einer geraden Mittelachse die durch das gesamte Winkelstück bis zum Obertrieb im Kopf des Winkelstücks verläuft, entwickelt werden, ohne dabei auf die Biegung der Außenhülse verzichten zu müssen. Die Welle und gegebenenfalls der Mitnehmer als funktioneller Teil der Welle sind hierbei am proximalen Ende und am distalen Ende der Außenhülse im Wesentlichen zentrisch angeordnet, wo hingegen sie in einem Abschnitt der Außenhülse zwischen dem proximalen Ende und distalen Ende exzentrisch angeordnet ist. Die Biegung der Außenhülse des Winkelstücks beträgt bevorzugt etwa 8°-16°, besonders bevorzugt 10°-14°.

In umfangreichen Praxisversuchen konnte bemerkenswerter Weise festgestellt werden, daß ein derartiges erfindungsgemäßes Winkelstück dem Anwender die gleichen Vorteile in Bezug auf Ergonomie und freie Sicht auf die Behandlungsstelle bietet, wie aus dem Stand der Technik bekannte Winkelstücke. Gleichzeitig konnte jedoch eine Reduktion der Lärmemission und eine verbesserte Übertragung des Drehmoments mit geringeren Verlusten ermittelt werden. Zusätzlich erlaubt das erfindungsgemäße Winkelstück eine Reduktion des Herstellungs- und Montageaufwands.

In einem bevorzugten Ausführungsbeispiel schließen die Mittelachse des Halsteils der Außenhülse und die Mittelachse des Kopfs einen Winkel von größer / gleich 90°, bevorzugt von 92° - 95° ein, wodurch die Handhabung des Winkelstücks für den Anwender noch komfortabler wird.

Die Erfindung wird nachfolgend anhand bevorzugter Ausführungsbeispiele und Bezug nehmend auf die beigefügten Zeichnungen erläutert:
Figur 1 zeigt ein erstes Ausführungsbeispiel eines erfindungsgemäßen Winkelstück.
Figur 2 zeigt ein zweites Ausführungsbeispiel eines erfindungsgemäßen Winkelstück.

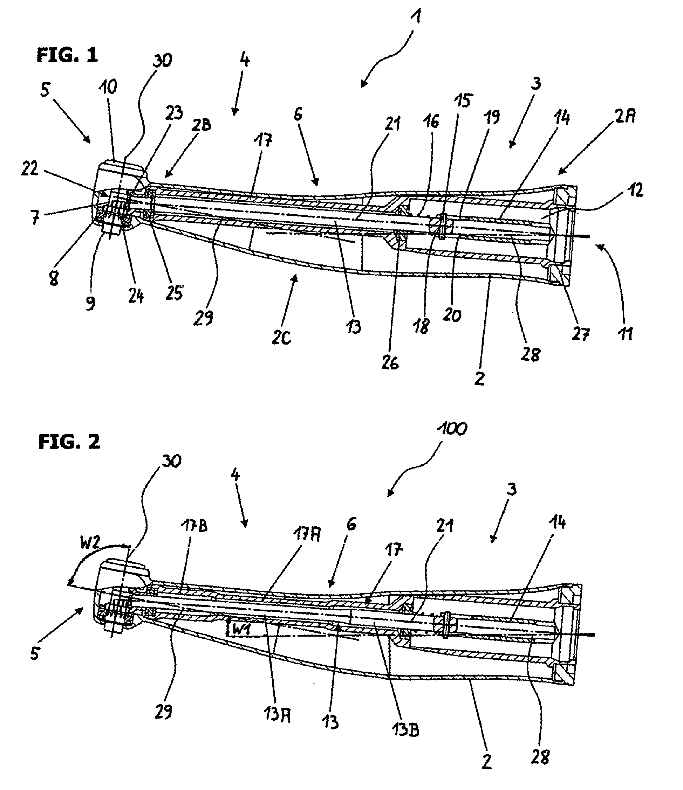

Die Außenhülse 2 des in Figur 1 dargestellten Winkelstücks 1 besteht aus einem Griffteil 3. einem Halsteil 4 und dem Winkelstückkopf 5. Im Übergangsbereich von Griffteil 3 zu Halsteil 4 weist die Außenhülse 2 eine Biegung 6 auf, die dem Anwender ergonomische Vorteile bringt und eine verbesserte Sicht auf die Behandlungsstelle ermöglicht.

Im Winkelstückkopf 5 ist In bekannter Weise eine Werkzeugaufnahme 7 als Teil der Welle 22 (Obertrieb) angeordnet, die durch zwei Lager, bevorzugt Wälzlager 8, gelagert wird (aufgrund der nur teilweise entfernten Außenhülse ist nur ein Lager sichtbar). Die Werkzeugaufnahme 7 weist auf einer Seite eine Öffnung 9 zum Einstecken eines Behandlungswerkzeugs, zum Beispiel eines Bohrers (nicht dargesteollt), auf. Auf der der Öffnung 9 gegenüberliegenden Seite des Kopfs 5 befindet sich ein Druckknopf 10 zum Lösen des Behandlungswerkzeugs aus der Werkzeugaufnahme 7.

Am proximalen Ende 2A der Außenhülse 2 ist eine Kupplungsvorrichtung 11 zum Anschluß einer Antriebseinheit, bevorzugt eines E-Motors, vorgesehen Zur Übertragung der Antriebsbewegung vom Motor auf das Behandlungswerkzeug wird in bekannter Weise ein am distalen Ende der Antriebseinheit angeordneter Kupplungszapfen (nicht dargestellt) in die Aufnahme 12 des Winkelstücks 1 eingeführt, wodurch die Motorwelle des Motors mit der Welle 13 des Winkelstücks 1 verbunden wird. Aufnahme 12 wird durch eine Vergrößerung der lichten Weite der Lagerhülse 17 in ihrem proximalen Abschnitt gebildet.

Am proximalen Ende der Welle 13 ist eine Querbohrung 18 zur Aufnahme eines Zylinderkerbstifts 15 vorgesehen. Mit Hilfe des Zylinderkerbstifts 15 wird Mitnehmer 14 mit der Welle 13 verbunden. Mitnehmer 14 weist zwei Längsschlitze 19, 20 auf, durch die Stift 15 ragt. Feder 16 spannt den Mitnehmer 14 gegenüber Welle 13 und der Lagerhülse 17 vor. Aufgrund der beiden Längsschlitze 19, 20 ist Mitnehmer 14 parallel zur Mittelachse 21 der Welle 13 verschiebbar. Dieses Spiel gewährleistet eine sichere Kupplung zwischen der Antriebseinheit und dem Winkelstück 1 und eine zuverlässige Übertragung der Rotationsbewegung und des Drehmoments von der Rotorwelle und auf die Welle 13.

Erfindungsgemäß erfolgt die Übertragung der Antriebsbewegung durch das gesamte Winkelstück 1 bis zum Obertrieb 22 im Kopf 5 durch eine einzige, einstückige, starre Welle 13. Welle 13 ist dabei so im Winkelstück 1 angeordnet, daß sie, bzw. der Mitnehmer 14 als Teil und funktionelle Verlängerung der Welle 13, am proximalen Ende 2A und am distalen Ende 2 B der Außenhülse 2 im wesentlichen zentrisch angeordnet ist, wo hingegen in einem Abschnitt 2 C der Außenhülse 2 zwischen dem proximalen Ende 2 A und distalen Ende 2 B exzentrisch angeordnet ist. Am distalen Ende der Welle 13 befindet sich ein Zahnrad 23, das mit einem weiteren Zahnrad 24 des Obertriebs 22 kämmt und die Antriebsbewegung auf den Obertrieb 22 und das in der Werkzeugaufnahme 7 fixierte Werkzeug überträgt.

Welle 13 ist in der ebenfalls einstückig ausgeführten Lagerhülse 17 mittels zweier Wälzlager 25, 26 gelagert. Lagerhülse 17 ist mit ihrem distalen Ende an der Innenwand der Außenhülse 2 und mit ihrem proximalen Ende an einem Gewindering 27 als Teil der Kupplung 11 gelagert.

Das in Figur 2 dargestellt erfindungsgemäße Winkelstück 100 entspricht im Aufbau dem Winkelstück 1 aus Figur 1, so daß zur Venneidung von Wiederholungen von einer detaillierten Beschreibung abgesehen wird.

Im Unterschied zu Figur 1 weisen die Welle 13 und die Lagerhülse 17 des Winkelstücks 100 jeweils mehrere Abschnitte 13 A und 13 B bzw. 17 A und 17 B mit unterschiedlichen Durchmessern auf. Der Abschnitt 17 A mit dem geringeren Durchmesser umgibt dabei zumindest Teile des Abschnitts 13 A mit dem geringeren Durchmesser. Die jeweiligen Abschnitte 13 A und 17 A mit den geringeren Durchmessern sich im Bereich der Biegung 6 der Außenhülse 2, wodurch in bevorzugter Weise eine Biegung der Außenhülse 2 erreicht werden kann. Selbstverständlich können die Welle 13 und die Lagerhülse 17 auch mehr als zwei Abschnitte mit unterschiedlichen Durchmessern umfassen.

Zusätzlich sind in Figur 2 auch noch Angaben zu den relevanten Winkel zwischen den einzelnen Bauteilen des Winkelstücks 100 angeführt, die in gleicher Weise auch für das Winkelstück 1 in Figur 1 gelten.

Die Mittelachse 28 des Griffteils 3 und die Mittelachse 29 des Halsteils 4 schließen einen Winkel W1 ein, der im Bereich von 8°-16°, bevorzugt 10°-14° liegt. Winkel W1 ist jener Winkel, der die für den Anwender wichtige Biegung 6 beschreibt. Winkel W2 wird durch die Mittelachse 29 des Halsteils 4 und die Mittelachse 30 des Kopfs 5 gebildet. Winkel w2 ist größer / gleich 90°, bevorzugt von 92° - 95°. Die Neigung des Kopfs 5 gegenüber dem Halsteil 4 um den Winkel W2 ergänzt die Biegung 6 durch den Winkel W1, so daß in Summe die erfindungsgemäßen Winkelstücke 1 und 100 dem Anwender die gleichen Vorteile in Bezug auf Ergonomie und freie Sicht auf die Behandlungsstelle bieten, wie aus dem Stand der Technik bekannte Winkelstücke.

Die Erfindung ist nicht auf das dargestellte Anwendungsgebiet und das beschriebene Ausführungsbeispiel beschränkt. So umfaßt insbesondere der Begriff "einstückige Welle" auch Wellen, die aus mehreren bestehen und die bevorzugt während der Montage des Winkelstücks miteinander verbunden, zum Beispiel durch eine Formschlußverbindung, insbesondere durch eine chraubverbindung, so daß bei Betrieb des Winkelstücks eine funktionell einstückige Welle ohne zwischengeschaltete Zahnräder oder Getriebe vorliegt.

## Patentansprüche

1. Medizinisches Winkelstück (1, 100) zum Antrieb eines mit dem Winkelstück (1, 100) verbundenen Werkzeugs mit einer Außenhülse (2), die einen Griffteil (3), einen Halsteil (4) mit einer Mittelachse (29) und einen Winkelstückkopf (5) umfasst, wobei die Außenhülse (2) im Übergangsbereich zwischen Griffteil (3) und Halsteil (4) eine Biegung (6) aufweist, mit einer im Winkelstückkopf (5) angeordneten Welle (22) mit einer Werkzeugaufnahme (7), mit einer einstückigen, den Griffteil (3), die Biegung (6) und den Halsteil (4) durchsetzenden Welle (13) zur Übertragung der Antriebsbewegung von einer Antriebseinheit auf die Werkzeugaufnahme (7) oder mit einer funktionell einstückigen Welle, die aus mehreren Teilen (13, 14) besteht, die miteinander verbunden sind, so daß bei Betrieb des Winkelstücks (1, 100) eine funktionell einstückige Welle ohne zwischengeschaltete Zahnräder oder Getriebe vorliegt, und mit einer sich durch den Griffteil (3), die Biegung (6) und den Halsteil (4) erstreckenden Mittelachse (21) der einstückigen Welle (13) oder der funktionell einstückigen Welle (13, 14), **dadurch gekennzeichnet, daß** die Mittelachse (21) der einstückigen Welle (13) oder der Funktionell einstückigen Welle (13, 14) gerade ist und die Mittelachse (29) des Halsteils (4) und die Mittelachse (21) der einstückigen Welle (13) oder der Funktionell einstückigen Welle (13, 14) einen Winkel einschließen.

2. Winkelstück (1, 100) nach Anspruch 1, **dadurch gekennzeichnet, daß** die einstückige Welle (13) oder die funktionell einstückige Welle (13, 14) am proximalen Ende (2 A) und am distalen Ende (2 B) der Außenhülse (2) im wesentlichen zentrisch angeordnet ist, wo hingegen sie in einem Abschnitt (2 C) der Außenhülse (2) zwischen dem proximalen Ende (2 A) und dem distalen Ende (2 B) exzentrisch angeordnet ist.

3. Winkelstück (1, 100) nach Anspruch 1 oder 2, **dadurch gekennzeichnet, daß** der von der Mittelachse (28) des Griffteils (3) und der Mittelachse (29) des Halsteils (4) eingeschlossene Winkel (W1) 8°-16°, bevorzugt 10°-14° beträgt.

4. Winkelstück (1, 100) nach einem der vorhergehenden Ansprüche, **dadurch gekennzeichnet, daß**
die einstückige Welle (13) oder die funktionell einstückige Welle (13, 14) einen ersten und einen zweiten Durchmesser (13 A, 13 B) aufweist.

5. Winkelstück (1, 100) nach Anspruch 4, **dadurch gekennzeichnet, daß** der zweite Durchmesser (13 A) geringer ist als der erste Durchmesser (13 B) und daß sich der Abschnitt mit dem zweiten Durchmesser (13 A) im Bereich der Biegung (6) der Außenhülse (2) befindet.

6. Winkelstück (1, 100) nach Anspruch 4 oder 5, **dadurch gekennzeichnet, daß** die einstückige Welle (13) oder die funktionell einstückige Welle (13, 14) in einer Lagerhülse (17) angeordnet ist, die Lagerhülse (17) ebenfalls einen ersten und einen zweiten, geringeren Durchmesser (17 A, 17 B) aufweist und der Abschnitt der Lagerhülse (17) mit dem geringeren Durchmesser (17 A) zumindest Teile des Abschnitts der einstückigen Welle (13) oder der funktionell einstückigen Welle (13, 14) mit dem geringeren Durchmesser (13 A) umgibt.

7. Winkelstück (1, 100) nach einem der vorhergehenden Ansprüche, **dadurch gekennzeichnet, daß**
die Mittelachse (29) des Halsteils (4) der Außenhülse (2) und die Mittelachse (30) des Kopfs (5) einen Winkel (W2) von größer oder gleich 90°, bevorzugt von 92° - 95° einschließen.

8. Winkelstück (1, 100) nach einem der vorhergehenden Ansprüche, **gekennzeichnet durch** eine starre einstückige Welle (13) oder funktionell einstückige Welle (13, 14).

9. Winkelstück (1, 100) nach einem der vorhergehenden Ansprüche, **dadurch gekennzeichnet, daß**
die Teile (13, 14) der funktionell einstückigen Welle durch eine Formschlussverbindung, insbesondere durch eine Schraubverbindung, miteinander verbunden sind.

10. Winkelstück (1, 100) nach einem der vorhergehenden Ansprüche, **gekennzeichnet durch** eine am proximalen Ende der einstückigen Welle (13) oder der funktionell einstückigen Welle vorgesehene Querbohrung (18) zur Aufnahme eines Zylinderkerbstifts (15), welcher einen Mitnehmer (14) mit der einstückigen Welle (13) oder der funktionell einstückigen Welle verbindet, wobei der Mitnehmer (14) zwei Längsschlitze (19, 20) aufweist, **durch** die der Stift (15) ragt.

11. Winkelstück (1, 100) nach Anspruch 10, **gekennzeichnet durch**
eine Feder (16), die den Mitnehmer (14) gegenüber der einstückigen Welle (13) oder der funktionell einstückigen Welle (13, 14) und der Lagerhülse (17) vorspannt.

12. Winkelstück (1, 100) nach einem der vorhergehenden Ansprüche, **dadurch gekennzeichnet, daß**
die im Winkelstückkopf (5) angeordnete Welle (22) mit einer Werkzeugaufnahme (7) durch zwei Lager, bevorzugt Wälzlager (8), gelagert ist.

13. Winkelstück (1, 100) nach einem der vorhergehenden Ansprüche, **dadurch gekennzeichnet, daß**
die Werkzeugaufnahme (7) an einer Seite eine Öffnung (9) zum Einstecken eines Behandlungswerkzeugs, zum Beispiel eines Bohrers, aufweist und auf der der Öffnung (9) gegenüberliegenden Seite des Winkelstückkopfs (5) sich ein Druckknopf (10) zum Lösen des Behandlungswerkzeugs aus der Werkzeugaufnahme (7) befindet.

14. Winkelstück (1, 100) nach einem der vorhergehenden Ansprüche, **gekennzeichnet durch** eine Zahnrad (23) am distalen Ende der einstückigen Welle (13) oder der funktionell einstückigen Welle (13, 14), das mit einem weiteren Zahnrad (24) der im Winkelstückkopf (5) angeordneten Welle (22) kämmt, um die Antriebsbewegung auf die im Winkelstückkopf (5) angeordneten Welle (22) und das in der Werkzeugaufnahme (7) fixierte Werkzeug zu übertragen.

15. Winkelstück (1, 100) nach einem der vorhergehenden Ansprüche, **dadurch gekennzeichnet, daß**
die einstückige Welle (13) oder die funktionell einstückige Welle (13, 14) in einer einstückig ausgeführten Lagerhülse (17) mittels zweier Wälzlager (25, 26) gelagert ist.

## Claims

1. Medical contra-angle handpiece (1, 100) for driving a tool connected to the contra-angle handpiece (1, 100) with an outer sleeve (2) comprising a handle section (3), a neck section (4) with a center axis (29) and a contra-angle handpiece head (5), wherein the outer sleeve (2) comprises a curvature (6) in the region of transition between the handle section (3) and the neck section (4), with a shaft (22) with a tool holder (7) arranged in the contra-angle handpiece head (5), with a single-piece shaft (13) for the transmission of a drive movement from a drive unit to the tool holder (7) extending through the handle section (3), the curvature (6) and the neck section (4) or with a functionally single-piece shaft, which consists of several parts (13, 14), which are connected with each other, so that during operation of the contra-angle handpiece (1, 100) a functionally single-piece shaft without interconnected gear wheels or transmissions is achieved, and with a center axis (21) of the single-piece shaft (13) or the functionally single-piece shaft (13, 14) extending through the handle section (3), the curvature (6) and the neck section (4), **characterized in that** the center axis (21) of the single-piece shaft (13) or of the functionally single-piece shaft (13, 14) is straight and the center axis (29) of the neck section (4) and the center axis (21) of the single-piece shaft (13) or of the functionally single-piece shaft (13, 14) enclose an angle.

2. Contra-angle handpiece (1, 100) in accordance with Claim 1, **characterized in that** the single-piece shaft (13) or the functionally single-piece shaft (13, 14) are essentially centered at the proximal end (2A) and the distal end (2B) of the outer sleeve (2), whereas they are arranged eccentrically in a section (2C) of the outer sleeve (2) between the proximal end (2A) and the distal end (2B).

3. Contra-angle handpiece (1, 100) in accordance with Claim 1 or 2, **characterized in that** the angle (W1) between the center axis (28) of the handle section (3) and the center axis (29) of the neck section (4) is in the range of 8°-16°, preferably 10°-14°.

4. Contra-angle handpiece (1, 100) in accordance with one of the preceding claims, **characterized in that**
the single-piece shaft (13) or the functionally single-piece shaft (13, 14) have a first and second diameter (13A, 13B).

5. Contra-angle handpiece (1, 100) in accordance with Claim 4, **characterized in that** the second diameter (13A) is smaller than the first diameter (13B) and that the section having the second diameter (13A) is in the region of curvature (6) of the outer sleeve (2).

6. Contra-angle handpiece (1, 100) in accordance with Claim 4 or 5, **characterized in that** the single-piece shaft (13) or the functionally single-piece shaft (13, 14) are arranged in a bearing sleeve (17), wherein the bearing sleeve (17) also has a first and a second, smaller,
diameter (17A, 17B), with the section of the bearing sleeve (17) having the smaller diameter (17A) surrounding at least parts of the single-piece shaft (13) or of the functionally single-piece shaft (13, 14) with the smaller diameter (13A).

7. Contra-angle handpiece (1, 100) in accordance with one of the preceding claims, **characterized in that**
the center axis (29) of the neck section (4) of the outer sleeve (2) and the center axis (30) of the head (5) enclose an angle (W2) greater than or equal to 90°, preferably in the range of 92° - 95°.

8. Contra-angle handpiece (1, 100) in accordance with one of the preceding claims, **characterized by**
a rigid single-piece shaft (13) or functionally single-piece shaft (13, 14).

9. Contra-angle handpiece (1, 100) in accordance with one of the preceding claims, **characterized in that**
the parts (13, 14) of the functionally single-piece shaft are connected by a form-fitting connection, in particular by a screw connection.

10. Contra-angle handpiece (1, 100) in accordance with one of the preceding claims, **characterized by**
a transverse bore (18) at the proximal end of the single-piece shaft (13) or the functionally single-piece shaft (13, 14) for holding a grooved cylindrical pin (15), which connects a dog (14) to the single-piece shaft (13) or to the functionally single-piece shaft (13, 14), wherein the dog (14) has two longitudinal slits (19, 20) for passing through the pin (15).

11. Contra-angle handpiece (1, 100) in accordance with Claim 10, **characterized by** a spring (16) which preloads the dog (14) toward the single-piece shaft (13) or the functionally single-piece shaft (13, 14) and the bearing sleeve (17).

12. Contra-angle handpiece (1, 100) in accordance with one of the preceding claims, **characterized in that**
the shaft (22) with a tool holder (7) arranged in the contra-angle handpiece head (5) is supported by two bearings, preferably roller bearings (8).

13. Contra-angle handpiece (1, 100) in accordance with one of the preceding claims, **characterized in that**
the tool holder (7) comprises an opening (9) on one side for inserting a treatment tool, for example a drill and on the side of the contra-angle handpiece head (5) opposite the opening (9) there is a pushbutton (10) for releasing the treatment tool from the instrument carrier (7).

14. Contra-angle handpiece (1, 100) in accordance with one of the preceding claims, **characterized by**
a gearwheel (23) at the distal end of the single-piece shaft (13) or the functionally single-piece shaft (13, 14), which meshes with a gearwheel (24) of the shaft (22) arranged in the contra-angle handpiece head (5) to transmit the drive movement to the shaft (22) arranged in the contra-angle handpiece head (5) and to the tool fixed in the tool holder (7).

15. Contra-angle handpiece (1, 100) in accordance with one of the preceding claims, **characterized in that**
the single-piece shaft (13) or the functionally single-piece shaft (13, 14) are supported in a single-piece bearing sleeve (17) by means of two roller bearings (25, 26).

## Revendications

1. Pièce angulaire médicale (1, 100) destinée à entraîner un outil relié à la pièce angulaire (1, 100) comprenant un manchon extérieur (2) qui comprend une partie de poignée (3), une partie de collet (4) avec un axe médian (29) et une tête de pièce angulaire (5), sachant que le manchon extérieur (2) présente une flexion (6) au niveau du passage entre la partie de poignée (3) et la partie de collet (4), avec un arbre (22) disposé dans la tête de pièce angulaire (5) avec une réception d'outil (7), avec un arbre monobloc (13) traversant la partie de poignée (3), la flexion (6) et la partie de collet (4) pour transmettre le mouvement d'entraînement d'une unité d'entraînement à la réception d'outil (7) ou avec un arbre monobloc fonctionnel composé de plusieurs pièces (13, 14) reliées entre elles, de façon à ce que lors de l'utilisation de la pièce angulaire (1, 100), un arbre monobloc fonctionnel soit présent sans roues dentées ou transmissions intermédiaires, et avec un axe médian (21) de l'arbre monobloc (13) ou de l'arbre monobloc fonctionnel (13, 14) s'étendant à travers la partie de poignée (3), la flexion (6) et la partie de collet (4), **caractérisée en ce que** l'axe médian (21) de l'arbre monobloc (13) ou de l'arbre monobloc fonctionnel (13, 14) est droit et que l'axe médian (29) de la partie de collet (4) et l'axe médian (21) de l'arbre monobloc (13) ou de l'arbre monobloc fonctionnel (13, 14) délimitent un angle.

2. Pièce angulaire (1, 100) selon la revendication 1, **caractérisée en ce que** l'arbre monobloc (13) ou de l'arbre monobloc fonctionnel (13, 14) est disposé sur l'extrémité proximale (2 A) et sur l'extrémité distale (2 B) du manchon extérieur (2) essentiellement de manière centrique, alors qu'il est disposé de manière excentrique dans un tronçon (2 C) du manchon extérieur (2) entre l'extrémité proximale (2 A) et l'extrémité distale (2 B).

3. Pièce angulaire (1, 100) selon la revendication 1 ou 2, **caractérisée en ce que** l'angle (W1) compris par l'axe médian (28) de la partie de poignée (3) et l'axe médian (29) de la partie de collet (4) est de 8°-16°, de préférence de 10°-14°.

4. Pièce angulaire (1, 100) selon l'une des revendications précédentes, **caractérisée en ce que** l'arbre monobloc (13) ou l'arbre monobloc fonctionnel (13, 14) présente un premier et un second diamètre (13 A, 13 B).

5. Pièce angulaire (1, 100) selon la revendication 4, **caractérisée en ce que** le second diamètre (13 A) est plus petit que le premier diamètre (13 B) et que le tronçon avec le second diamètre (13 A) se trouve au niveau de la flexion (6) du manchon extérieur (2).

6. Pièce angulaire (1, 100) selon la revendication 4 ou 5, **caractérisée en ce que** l'arbre monobloc (13) ou l'arbre monobloc fonctionnel (13, 14) est disposé dans un manchon de palier (17), le manchon de palier (17) présente également un premier et un second diamètre plus petit (17 A, 17 B) et le tronçon du manchon de palier (17) avec le diamètre plus petit (17 A) entoure au moins des parties du tronçon de l'arbre monobloc (13) ou de l'arbre monobloc fonctionnel (13, 14) avec le diamètre plus petit (13 A).

7. Pièce angulaire (1, 100) selon l'une des revendications précédentes, **caractérisée en ce que** l'axe médian (29) de la partie de collet (4) du manchon extérieur (2) et l'axe médian (30) de la tête (5) délimitent un angle (W2) supérieur ou égal à 90°, de préférence de 92° - 95°.

8. Pièce angulaire (1, 100) selon l'une des revendications précédentes, **caractérisée par** un arbre monobloc (13) rigide ou un arbre monobloc fonctionnel (13, 14) rigide.

9. Pièce angulaire (1, 100) selon l'une des revendications précédentes, **caractérisée en ce que** les pièces (13, 14) de l'arbre monobloc fonctionnel sont reliées entre elles par une liaison par complémentarité de forme, en particulier par un assemblage par vis.

10. Pièce angulaire (1, 100) selon l'une des revendications précédentes , **caractérisée par** un alésage transversal (18) prévu sur l'extrémité proximale de l'arbre monobloc (13) ou de l'arbre monobloc fonctionnel pour recevoir une goupille cannelée de cylindre (15), laquelle relie un entraîneur (14) à l'arbre monobloc (13) ou à l'arbre monobloc fonctionnel, sachant que l'entraîneur (14) présente deux fentes longitudinales (19, 20) à travers lesquelles la goupille (15) fait saillie.

11. Pièce angulaire (1, 100) selon la revendication 10, **caractérisée par**
un ressort (16) qui précontraint l'entraîneur (14) par rapport à l'arbre monobloc (13) ou l'arbre monobloc fonctionnel (13, 14) et le manchon de palier (17).

12. Pièce angulaire (1, 100) selon l'une des revendications précédentes, **caractérisée en ce que** l'arbre (22) comprenant une réception d'outil (7) disposé dans la tête de pièce angulaire (5) est monté sur deux paliers, de préférence des paliers à roulement (8).

13. Pièce angulaire (1, 100) selon l'une des revendications précédentes, **caractérisée en ce que** la réception d'outil (7) présente sur un côté une ouverture (9) pour enficher un outil de traitement, par exemple un outil à percer, et sur le côté de la tête de pièce angulaire (5) faisant face à l'ouverture (9) se trouve un bouton poussoir (10) pour désolidariser l'outil de traitement de la réception d'outil (7).

14. Pièce angulaire (1, 100) selon l'une des revendications précédentes, **caractérisée par** une roue dentée (23) sur l'extrémité distale de l'arbre monobloc (13) ou de l'arbre monobloc fonctionnel (13, 14) qui s'engrène avec une autre roue dentée (24) de l'arbre (22) disposé dans la tête de pièce angulaire (5) pour transmettre le mouvement d'entraînement à l'arbre (22) disposé dans la tête de pièce angulaire (5) et à l'outil fixé dans la réception d'outil (7).

15. Pièce angulaire (1, 100) selon l'une des revendications précédentes, **caractérisée en ce que** l'arbre monobloc (13) ou l'arbre monobloc fonctionnel (13, 14) est monté sur un manchon de palier (17) réalisé de façon monobloc, via deux paliers à roulement (25, 26).
